Europäisches Patentamt

European Patent Office

Office européen des brevets

(19)

(11) EP 0 755 230 B1

(12) **EUROPÄISCHE PATENTSCHRIFT**

(45) Veröffentlichungstag und Bekanntmachung des Hinweises auf die Patenterteilung:
**14.01.1998 Patentblatt 1998/03**

(21) Anmeldenummer: 95915058.2

(22) Anmeldetag: **10.04.1995**

(51) Int. Cl.$^6$: **A61C 1/00**

(86) Internationale Anmeldenummer:
**PCT/AT95/00073**

(87) Internationale Veröffentlichungsnummer:
**WO 95/27446** (19.10.1995 Gazette 1995/45)

(54) **VERFAHREN ZUR GEWEBEUNTERSCHEIDUNG SOWIE VORRICHTUNG ZUR BEARBEITUNG VON ZAHNHARTGEWEBE MIT LASERIMPULSEN**

TISSUE-DIFFERENTIATING PROCESS AND DEVICE FOR TREATING HARD DENTAL TISSUE BY LASER PULSES

PROCEDE DE DIFFERENCIATION DE TISSUS ET DISPOSITIF POUR LE TRAITEMENT DE TISSUS DENTAIRES DURS PAR IMPULSIONS LASER

(84) Benannte Vertragsstaaten:
**AT DE FR GB**

(30) Priorität: **11.04.1994 RU 94012665**

(43) Veröffentlichungstag der Anmeldung:
**29.01.1997 Patentblatt 1997/05**

(73) Patentinhaber:
**LMS LASER MEDICAL SYSTEMS ERZEUGUNG UND VERTRIEB MEDIZINISCH TECHNISCHER GERATE GESELLSCHAFT M.B.H.
7000 Eisenstadt (AT)**

(72) Erfinder:
• **ALTSHULER, Gregory B.
St. Petersburg, 196240 (RU)**
• **EROFEEV, Andrey V.
St. Petersburg, 192242 (RU)**
• **BELIKOV, Andrey V.
St. Petersburg, 195272 (RU)**

(74) Vertreter:
**Pinter, Rudolf, Dipl.-Ing. et al
Patentanwälte Klein & Pinter OEG
Fasangasse 49
1030 Wien (AT)**

(56) Entgegenhaltungen:
**WO-A-90/04358          WO-A-90/07904
WO-A-92/16154          DE-A- 4 015 066
DE-C- 4 014 303**

**Beschreibung**

<u>Technisches Gebiet</u>

Die vorliegende Erfindung betrifft ein Verfahren zur Unterscheidung der Gewebearten bzw. zur Regelung der Strahlungsenergie bei der Bearbeitung von Zahnhartgewebe mit Laserimpulsen. Weiters betrifft die Erfindung auch eine entsprechende Vorrichtung mit einem Impulslaser und einer eingangsseitig mit dem Laseraustritt in Verbindung stehenden Strahlungstransporteinrichtung.

<u>Stand der Technik</u>

Bekannt sind beispielsweise aus US-A-4,521.194 Verfahren bzw. Vorrichtungen zur Entfernung beginnender kariöser Schädigungen bzw. von Zahnstein, die die Bearbeitung von Zahnhartgewebe mit gepulster Laserstrahlung bzw. entsprechenden Lasern beinhalten. Weiters ist beispielsweise aus dem Urheberschein UdSSR Nr. 1,593.669 ein Verfahren zur Behandlung von nichtkomplizierter Karies bekannt, welches die Bearbeitung von Zahnhartgewebe mit Laserstrahlung mit einer Wellenlänge von 2,94 Mikrometer, mit einer Impulsdauer von 100 - 500 Mikrosekunden und einer Leistung von 0,5 - 1,0 Joule/Impuls, einer Leistungsdichte von $2.10^4 \pm 3.10$ W/cm$^2$, einer Frequenz von 1 Hz und einer Expositionsdauer von 3 - 30 s beinhaltet.

Aus WO 89/08432 ist beispielsweise eine Vorrichtung zur Bearbeitung von Zahnhartgewebe mit Laserstrahlung bekannt, die hintereinander einen entlang der optischen Achse angeordneten Impulslaser und ein Strahlungstransportmittel zum Zahn in Form einer optischen Faser aufweist. Beispielsweise aus WO 90/01907 ist auch eine Vorrichtung zur Bearbeitung von Zahnhartgewebe mit Laserstrahlung bekannt, die hintereinander einen entlang der optischen Achse angeordneten Impulslaser und ein Strahlungstransportmittel zum Zahn umfaßt, das seinerseits ein Stück optische Faser, deren Eintritt optisch mit dem Austritt des Lasers verknüpft ist, und ein Handstück aufweist, dessen Eintritt optisch mit dem Austritt der optischen Faser verknüpft ist, wobei dieser Austritt den Austritt der Vorrichtung darstellt.

Der wesentlichste Mangel aller angeführten bekannten Vorrichtungen bzw. Verfahren besteht in der Gefahr, daß bei der Bearbeitung des Zahnhartgewebes ein Lasertrauma zugefügt werden kann, da die die Bearbeitung durchführende Person aus systemimmanenten Gründen keine Rückwirkung von der gerade bearbeiteten Stelle erhält, wie dies beispielsweise beim Umgang mit Zahnarztbohrern, Skalpellen oder dergleichen sehr wohl der Fall ist.

<u>Darstellung der Erfindung</u>

Die Aufgabe der vorliegenden Erfindung besteht im wesentlichen darin, bei einer Einrichtung bzw. einem Verfahren der eingangs genannten Art die beschriebene Gefahr, dem Patienten ein Lasertrauma zuzufügen, dadurch zu beseitigen bzw. zumindest zu verringern, daß eine Möglichkeit zur Bestimmung der Art des zu bearbeitenden Gewebes sichergestellt wird.

Die genannte Aufgabe wird dadurch gelöst, daß bei einem Verfahren zur Unterscheidung der Gewebearten bei der Bearbeitung von Zahnhartgewebe mit Laserimpulsen der bei der Wechselwirkung des Laserimpulses mit dem Gewebe entstehende akustische Impuls im Bereich von 85-95 kHz registriert und hinsichtlich seiner Spitzenamplitude überprüft wird, wonach aufgrund der Höhe der Spitzenamplitude die Art des jeweils bearbeiteten Gewebes bestimmt wird. Beim erfindungsgemäßen Verfahren zur Regelung der Strahlungsenergie bei der Bearbeitung von Zahnhartgewebe mit Laserimpulsen wird der bei der Wechselwirkung des Laserimpulses mit dem Gewebe entstehende akustische Impuls im Bereich von 85-95 kHz registriert und hinsichtlich seiner Spitzenamplitude überprüft, wonach aufgrund der Höhe der Spitzenamplitude die Art des jeweils bearbeiteten Gewebes bestimmt und die Strahlungsenergie der Gewebeart angepaßt wird.

Die erfindungsgemäße Vorrichtung zur Bearbeitung von Zahnhartgewebe mit Laserimpulsen, mit einem Impulslaser und einer eingangsseitig mit dem Laseraustritt in Verbindung stehenden Strahlungstransporteinrichtung, ist gemäß der Erfindung so ausgestattet, daß ein akustischer Empfänger in der Nähe des zu bearbeitenden Bereiches so angeordnet ist, daß die Richtung seiner maximalen Empfindlichkeit mit der Richtung der optischen Achse am Austritt der Strahlungstransporteinrichtung einen Winkel $\alpha > 11°$ und $< 86°$ bildet, daß der elektrische Signalausgang des Empfängers mit dem Eingang eines Spitzenamplitudenmessers verbunden ist, dessen Ausgang mit einer Anzeigevorrichtung in Verbindung steht, und daß dem Spitzenamplitudenmesser eine Einheit zur Spektralumwandlung und Filterung der vom akustischen Empfänger gelieferten elektrischen Signale vorgeschaltet ist, welche einen Durchlaßbereich von 85-95 kHz aufweist.

Im Zusammenhang mit der Einwirkung von Hochleistungslaserstrahlung auf eine abzutragende Substanz ist in anderen Zusammenhängen bekannt, daß akustische Wellen in der abzutragenden Substanz selbst sowie im Umgebungsmedium auftreten. Bekannt ist weiters, daß bestimmte Charakteristika dieser auftretenden Wellen von der Art, der Struktur und anderen Parametern der Substanz abhängen. Weiters ist bekannt, daß dieser Effekt grundsätzlich zur Unterscheidung der jeweils bearbeitenden Substanzart verwendet werden kann, wie` dies beispielsweise in WO-A-89/11829 bzw. auch WO-A-90/07904 für die Unterscheidung von Gefäßwänden gesunder Blutgefäße einerseits und karbonisierter Blutgefäße andererseits verwendet wird. Ähnliches ist auch bekannt zur Unterscheidung von Weichgewebe und Blasensteinen in der Harnblase sowie zur Unterscheidung von Weichgewebe und Knochengewebe in der Implantationschirurgie. Aus den als bekannt angeführten Beispielen ist die Verwendbarkeit von akustischen Signalen für die Unterscheidung oder Bestimmung der Materialart allerdings nicht für beliebige Substanzen und insbesonders nicht für Zahnhartgewebe herleitbar, da für den Fachmann erkenntlich völlig andere Material- und Umgebungsbedingungen am Einsatzort vorliegen.

Um die Zuverlässigkeit der Bestimmung der Art des bearbeiteten Gewebes zu erhöhen, kann in anderer Ausgestaltung der Erfindung gleichzeitig mit der Registrierung des akustischen Impulses auch die zeitliche Verzögerung zwischen dem Laserimpuls und dem akustischen Impuls gemessen werden, aus der der Typ des jeweils bearbeiteten Gewebes präzisiert werden kann.

Der vorher erwähnten vorteilhaften Ausgestaltung der erfindungsgemäßen Verfahren entspricht eine vorteilhafte Ausgestaltung der erfindungsgemäßen Vorrichtung, gemäß welcher am Laseraustritt eine Fotoregistriereinheit optisch eingekoppelt ist, deren elektrischer Signalausgang mit einem Eingang einer Intervallmeßeinheit verbunden ist, deren anderer Eingang mit dem Signalausgang des akustischen Empfängers verbunden ist und deren Ausgang mit der Anzeigevorrichtung in Verbindung steht.

Besonders bewährt hat sich im praktischen Einsatz der verschiedenen oben angesprochenen Verfahren nach der vorliegenden Erfindung eine Ausgestaltung, die dadurch gekennzeichnet ist, daß vor dem Beginn der Bearbeitung die den unterschiedlichen Gewebearten entsprechenden Spitzenamplituden und/oder Impulsverzögerungen an Kalibriermustern bekannter Gewebearten bestimmt und als Kalibrierwerte gespeichert werden, wonach die bei der tatsächlichen Bearbeitung bestimmten Spitzenwerte und/oder Impulsverzögerungen mit den gespeicherten Kalibrierwerten verglichen und demnach die Strahlungsenergie angepaßt wird.

Eine besonders bevorzugte weitere Ausgestaltung der erfindungsgemäßen Vorrichtung sieht schließlich vor, daß der akustische Empfänger in einem am Ausgang der Strahlungstransporteinrichtung angeordneten Handstück integriert ist, womit die Handhabung der Vorrichtung vereinfacht wird und stets gleichbleibende Abstands- und Winkelbedingungen für die Feststellung der akustischen Signale sichergestellt werden können.

<u>Beschreibung der Zeichnungen</u>

Die vorliegende Erfindung wird im folgenden noch anhand der vorwiegend schematischen Abbildungen näher erläutert. Es zeigen

Fig. 1a den akustischen Impuls, der bei der Laserzerstörung von Dentin auftritt,
Fig. 1b den akustischen Impuls, der bei der Laserzerstörung von Schmelz auftritt,
Fig. 1c die spektrale Abhängigkeit des Verhältnisses der akustischen Impulse, die bei der Laserzerstörung von Dentin einerseits und Schmelz andererseits auftreten,
Fig. 2a ein schematisches Zeitdiagramm, mit der Intensität des Laserimpulses, und
Fig. 2b mit der Energiedichte der Laserstrahlung, die auf die zu bearbeitende Oberfläche auftrifft,
Fig. 2c die Intensität der akustischen Impulse die bei der Zerstörung von Dentin und
Fig. 2d die bei der Zerstörung von Schmelz auftritt, und
Fig. 3 - 5 schematische Vorrichtungsanordnungen nach der Erfindung in unterschiedlicher Ausführung.

Zum Zahnhartgewebe zählen bekanntlich Schmelz und Dentin. Die Grenzwerte der Zerstörung dieser Gewebe durch Laserstrahlung unterscheiden sich beträchtlich, wobei die Zerstörungsschwelle von Dentin unter der Zerstörungsschwelle von Schmelz liegt. Bei der Bearbeitung von Zahnhartgewebe mit Laserstrahlung entsteht auf der zu bearbeitenden Oberfläche eine Erosionsflamme, die die visuelle Kontrolle des Zustandes der zu bestrahlenden Gewebeoberfläche und die Bestimmung des Gewebetyps unmöglich macht. Der Arzt ist mit anderen Worten nicht dazu imstande zu bestimmen, ob die Strahlung unmittelbar im Bearbeitungsprozeß auf den Schmelz oder auf das Dentin einwirkt. Dabei geht die Prozedur der Laserbearbeitung des Zahnhartgewebes mit der Gefahr einher, dem Dentin oder der Pulpa dann ein Lasertrauma zuzufügen, wenn die Impulsstrahlung mit einer Energie, die die Zerstörungsschwelle des Schmelzes übersteigt, auf das Dentin auftrifft. Der Hauptrisikofaktor ist die Gewebekontusion durch den Rückstoßimpuls, der dann entsteht, wenn die Strahlungsenergie, die auf das zu bearbeitende Gewebe auftritt, den Zerstörungsgrenzwert beträchtlich übersteigt.

Die von den Erfindern durchgeführten experimentellen Untersuchungen der Verfahrensweisen der Bearbeitung von Zahnhartgewebe (G.B. Altshuler, A.V. Belikov, A.V. Erofeev "The damage of hard tooth tissues with laser pulses of different duration", Proceeding 4th International Conference on Laser Application in Life Science, 1992, S. 114) ermöglichten es, einen hinsichtlich der Sicherheit der Prozedur der Laserbearbeitung des Zahns akzeptablen Bereich der Energiedichtewerte der Strahlung zu finden. Für Schmelz ist demnach zulässig, wenn die Energiedichte der Laserstrahlung die Zerstörungsschwelle um das zehnfache übersteigt. Für Dentin liegt der sichere Bereich im Rahmen der Hälfte dieses Wertes (d.h. zulässig ist nur, wenn die Energiedichte die Schwelle um das fünffache übersteigt), was sich durch die unmittelbare Nähe des Dentins zur Pulpa erklären läßt.

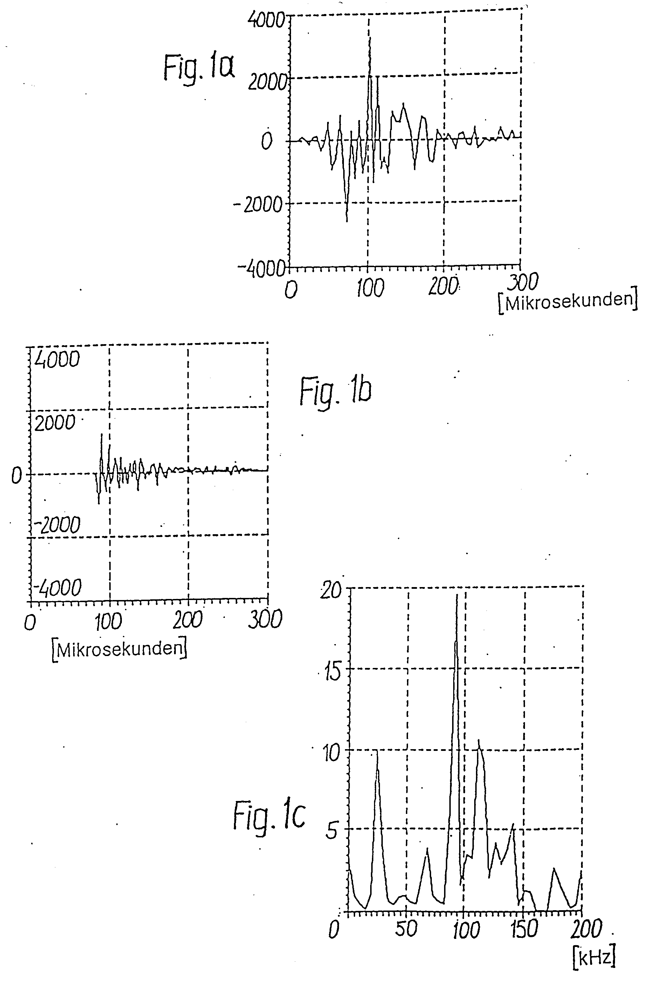

Um die Gefahr, dem Patienten bei der Bearbeitung von Zahnhartgewebe mit Laserstrahlung ein Trauma zuzufügen, zu verringern, wird vorgeschlagen, den Typ des zu bearbeitenden Gewebes (Schmelz, Dentin) nach den Charakteristika der akustischen Impulse zu identifizieren. Die Erfinder haben gefunden, daß sich die Spitzen(Maximal)amplitude des akustischen Impulses, der bei der Zerstörung von Dentin (Fig. 1a) entsteht, wesentlich (um ein Vielfaches) von der Spitzenamplitude des akustischen Impulses, der bei der Zerstörung von Schmelz (Fig. 1b) entsteht, unterscheidet. Auf diese Art und Weise ermöglicht die Einführung der Registrierung des akustischen Impulses, der bei der Wechselwirkung von Strahlung und Gewebe entsteht und auf Grund von dessen Spitzenamplitude man den Typ des zu bearbeitenden Gewebes bestimmen kann, im Verfahren zur Bearbeitung von Zahngewebe, falls erforderlich, die Strahlungsenergie zu senken und zu verhindern, daß dem Patienten ein Lasertrauma zugefügt wird.

Die Erfinder haben weiters ebenfalls gefunden, daß der wesentlichste Unterschied der Spitzenamplituden der akustischen Impulse, die bei der Zerstörung von Schmelz und Dentin auftreten, im Tonfrequenzband von 85 - 95 kHz zu beobachten ist. In Fig. 1c ist ein Diagramm des Verhältnisses der Amplituden der akustischen Impulsspektren, die im Dentin und im Schmelz erzeugt werden, dargestellt. Aus der Abbildung geht hervor, daß der Wert des Verhältnisses maximal vor allem im Tonfrequenzband von 85 - 95 kHz zu Tage tritt, wodurch die Spektralselektion der akustischen Impulse es ermöglicht, die Zuverlässigkeit der Bestimmung des Typs des zu bearbeitenden Gewebes zu erhöhen.

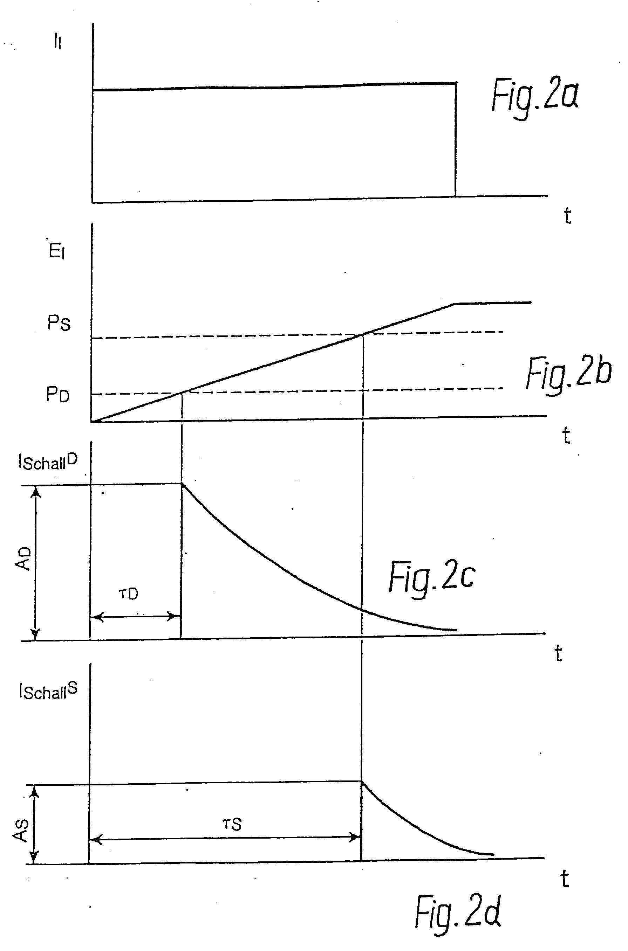

Bei der Einwirkung von gepulster Laserstrahlung, deren Energie den für die Zerstörung des Gewebes erforderlichen Wert übersteigt, auf das Zahngewebe läßt sich weiters eine zeitliche Verzögerung zwischen dem Beginn der Einwirkung des Laserimpulses und dem Einsetzen des akustischen Impulses, der vom Beginn der Gewebszerstörung zeugt, beobachten. Die Größe dieser Verzögerung wird durch drei Faktoren bestimmt: Durch die Entfernung der Tonquelle, die die zu bestrahlende Gewebsoberfläche darstellt, vom akustischen Empfänger, der den akustischen Impuls registriert, durch die Intensität der Laserstrahlung auf der Oberfläche des zu bearbeitenden Gewebes und durch den Schwellenwert der Gewebszerstörung (d.H. durch den Typ des zu bearbeitenden Gewebes). In Fig. 2 sind erklärende Zeitdiagramme über die Intensität des Laserimpulses (a), die Energiedichte der Laserstrahlung, die auf die zu bearbeitende Oberfläche auftrifft (b), sowie der akustischen Impulse, die bei der Zerstörung von Dentin (c) und Schmelz (d) entstehen, dargestellt. In Fig. 2b sind mit einer strichlierten Linie die den Zerstörungsschwellen von Schmelz und Dentin entsprechenden Energiedichteniveaus gezeigt. Aus den Fig. 2c,d geht hervor, daß der bei der Zerstörung von Dentin entstehende akustische Impuls eine geringere Verzögerung D hinsichtlich des Beginns des Laserimpulses als der bei der Zerstörung von Schmelz auftretende akustische Impuls ( S) aufweist. Auf diese Art und Weise ermöglicht es auch die Messung der Zeitverzögerungen der akustischen Impulse, den Typ des zu bearbeitenden Gewebes zu präzisieren.

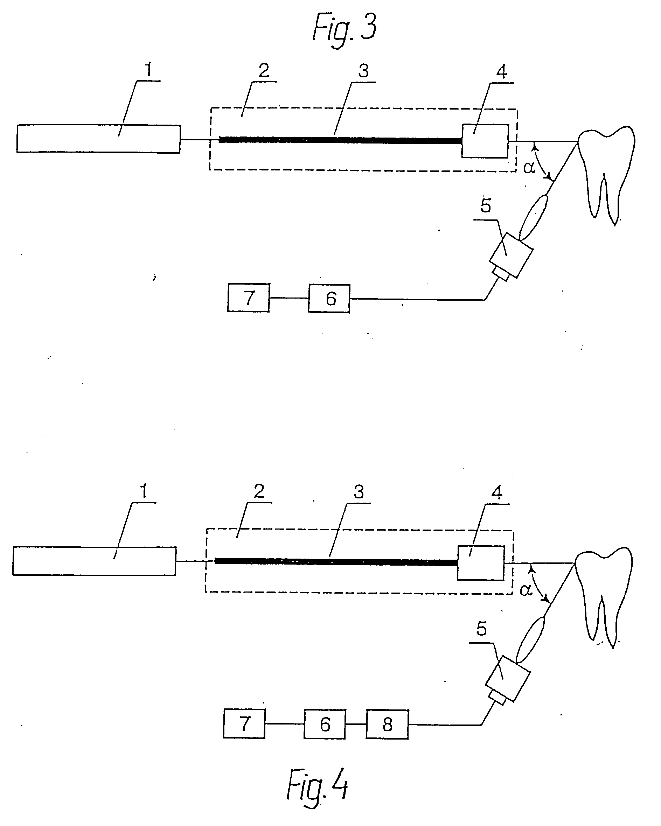

Ein Verfahren nach der Erfindung kann zum Beispiel mittels einer Vorrichtung, deren Schema in Fig. 3 vorgestellt wird, realisiert werden. In der Abbildung werden gezeigt der Laser 1, das Mittel 2 zum Transport der Laserstrahlung vom Laser zum Zahn, dessen Eintritt optisch mit dem Austritt des Lasers 1 verknüpft ist und das die optische Faser 3 und das Handstück 4, den akustischen Empfänger 5, der in der Weise installiert ist, daß die Richtung seiner maximalen Empfindlichkeit mit der Richtung der optischen Achse am Austritt des Strahlungstransportmittels vom Laser zum Zahn den Winkel $\alpha$ bildet, der die Bedingung: $11° < \alpha < 86°$ erfüllt, sowie den Spitzenamplitudenmesser 6 der elektrischen Impulse, dessen Eintritt elektrisch mit dem Austritt des akustischen Empfängers 5 verknüpft ist und dessen Austritt elektrisch mit dem Eintritt der Anzeigevorrichtung 7 verknüpft ist, beinhaltet.

Ein weiteres Verfahren nach der Erfindung kann zum Beispiel mittels einer Vorrichtung, deren Schema in Fig. 4 präsentiert wird, realisiert werden. In der Abbildung werden gezeigt der Laser 1, das Mittel 2 zum Transport der Laserstrahlung vom Laser zum Zahn, dessen Eintritt optisch mit dem Austritt des Lasers 1 verknüpft ist und das die optische Faser 3 und das Handstück 4, den akustischen Empfänger 5, der derart installiert ist, daß die Richtung seiner maximalen Empfindlichkeit mit der Richtung der optischen Achse am Austritt des Strahlungstransportmittels vom Laser zum Zahn den Winkel $\alpha$ bildet, der die Bedingung: $11° < \alpha < 86°$ erfüllt, sowie den Spitzenamplitudenmesser 6 der elektrischen Impulse, dessen Eintritt elektrisch durch die Einheit 8 zur Spektralumwandlung und Filterung mit dem Durchlaßbereich von 85 - 95 kHz mit dem Austritt des akustischen Empfängers 5 verknüpft ist und dessen Austritt elektrisch mit dem Eintritt der Anzeigevorrichtung 7 verknüpft ist, beinhaltet.

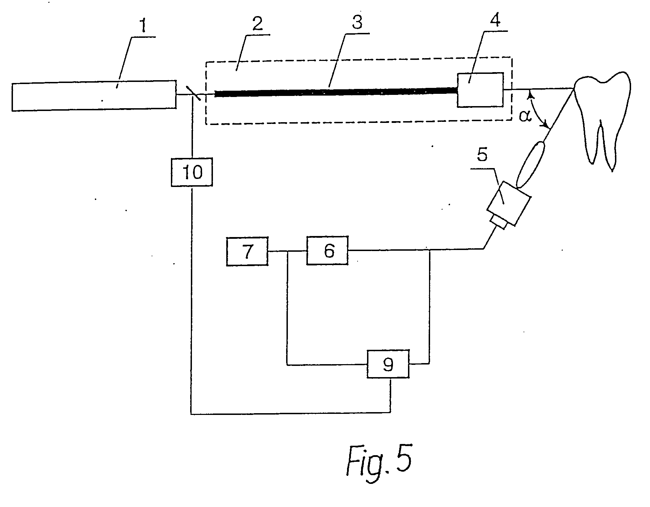

Ein anders ausgestaltetes Verfahren nach der Erfindung kann zum Beispiel mittels einer Vorrichtung, deren Schema in Fig. 5 präsentiert wird, realisiert werden. In der Abbildung werden dargestellt der Laser 1, das Mittel 2 zum Transport von Laserstrahlung vom Laser zum Zahn, dessen Eintritt optisch mit dem Austritt des Lasers 1 verknüpft ist und das die optische Faser 3 und das Handstück 4, den akustischen Empfänger 5, der in der Weise installiert ist, daß die Richtung seiner maximalen Emfpindlichkeit mit der Richtung der optischen Achse am Austritt des Strahlungstransportmittels vom Laser zum Zahn den Winkel $\alpha$ bildet, der die Bedingung: $11° < \alpha < 86°$ erfüllt, den Spitzenamplitudenmesser 6 der elektrischen Impulse, dessen Eintritt elektrisch mit dem Austritt des akustischen Empfängers 5 und dessen Austritt elektrisch mit dem Eintritt der Anzeigevorrichtung 7 verknüpft ist sowie die Einheit 9 zur Messung von Zeitintervallen und die Photoregistriereinheit 10 beinhaltet, wobei der Eintritt der Photoregistriereinheit 10 optisch mit dem Austritt des Lasers 1 verknüpft ist und deren Austritt elektrisch mit einem der Eintritte der Einheit 9 zur Messung von Zeitintervallen verknüpft ist, deren zweiter Eintritt elektrisch mit dem Austritt des akustischen Empfängers 5 verknüpft ist und dessen Austritt elektrisch mit dem Eintritt der Anzeigevorrichtung 7 verknüpft ist.

Ein Beispiel für die konkrete Realisierung eines erfindungsgemäßen Verfahrens wird wie folgt angeführt:

Vor Beginn der Bearbeitung des Zahnhartgewebes mit Laserstrahlung wird die Kalibrierung des Meßkanals der Vorrichtung durchgeführt. Dafür wird das Energieniveau der Erzeugung von Laser 1, das den der Zerstörungsschwelle des Schmelzes entsprechenden Wert übersteigt, festgestellt. Danach wird die vom Impulslaser erzeugte Strahlung durch das Transportmittel 2, das die optische Faser 3 und das Handstück 4 beinhaltet, auf die zu bearbeitende Oberfläche eines Kalibriermusters für Dentin gerichtet. Gleichzeitig damit wird mit der Photoregistrierungseinheit 10 der Laserimpuls registriert und in der Einheit 9 zur Messung von Zeitintervallen der Zeitpunkt fixiert, der seinem Beginn entspricht. Bei der Zerstörung von Dentin entsteht ein akustischer Impuls. Dieser akustische Impuls wird durch den sich nahe der zu bestrahlenden Zone befindlichen akustischen Empfänger 5 registriert. Mit dem Spitzenamplitudenmesser 6 wird die Spitzenamplitude des akustischen Impulses gemessen und der Zeitpunkt fixiert, der dem Beginn des akustischen Impulses entspricht. In der Einheit 9 zur Messung von Zeitintervallen wird die Verzögerung des Beginns des akustischen Impulses hinsichtlich des Beginns des Laserimpulses bestimmt. In der Einheit 8 zur Spektralumwandlung und Filterung erfolgt die Spektralumwandlung (zum Beispiel Fourier-Transformation) des akustischen Impulses und es wird die Spitzenamplitude des Spektrums des akustischen Impulses im Frequenzband von 85 bis 95 kHz gemessen. Die Werte der Spitzenamplitude, der Verzögerung und der Spitzenspektralamplitude (im angeführten Bereich) des bei der Zerstörung von Dentin entstehenden akustischen Impulses werden in der Anzeigevorrichtung 7 als Eichwerte für das Dentin fixiert.

Danach wird die vom Impulslaser erzeugte Strahlung durch das Transportmittel auf die zu bearbeitende Oberfläche eines Kalibriermusters für Schmelz gerichtet. Gleichzeitig damit wird der Laserimpuls registriert und der Zeitpunkt, der seinem Beginn entspricht, fixiert. Bei der Zerstörung von Schmelz entsteht ein akustischer Impuls. Dieser akustische Impuls wird durch den nahe der zu bestrahlenden Zone befindlichen akustischen Empfänger registriert. Die Spitzenamplitude des akustischen Impulses wird gemessen und der Zeitpunkt, der dem Beginn des akustischen Impulses entspricht, wird fixiert. Die Verzögerung des Beginns des akustischen Impulses hinsichtlich des Beginns des Laserimpulses wird bestimmt. Es erfolgt eine Spektralumwandlung (zum Beispiel Fourier-Transformation) des akustischen Impulses und die Spitzenamplitude des Spektrums des akustischen Impulses im Frequenzband von 85 bis 95 kHz wird gemessen. Die Werte der Spitzenamplitude, der Verzögerung und der Spitzenspektralamplitude (im angeführten Bereich) des akustischen Impulses, der bei der Zerstörung vom Schmelz entsteht, werden als Eichwerte für den Schmelz fixiert.

Nach dem Erhalt der Eichwerte für die Spitzenamplituden, die Verzögerungen und die Spitzenspektralamplituden der akustischen Impulse, die bei der Zerstörung von Schmelz und Dentin entstehen, wird die gepulste Laserstrahlung auf die zu bearbeitende Oberfläche des Zahnhartgewebes gerichtet. Der bei der Zerstörung des Zahnhartgewebes durch die Laserstrahlung entstehende akustische Impuls wird registriert. Die Spitzenamplitude dieses akustischen Impulses wird gemessen und mit den Eichwerten der Spitzenamplituden für Schmelz und Dentin verglichen. Wenn die gemessene Spitzenamplitude des akustischen Impulses A der Bedingung: $A < (A_S + A_D)/2$ erfüllt, wobei $A_S$ und $A_D$ die Eichwerte der Spitzenamplitude der akustischen Impulse darstellen, die bei der Laserzerstörung von Schmelz bzw. Dentin auftreten, so wird das zu bearbeitende Zahnhartgewebe als Schmelz und im gegenteiligen Falle als Dentin bestimmt.

Um die Zuverlässigkeit der Bestimmung des Typs des zu bearbeitenden Gewebes zu erhöhen, führt man eine Spektralumwandlung des akustischen Impulses durch und mißt den Spitzen(Maximal)wert der Amplitude des Spektrums des akustischen Impulses im Frequenzband von 85 bis 95 kHz. Man vergleicht diese mit den Eichwerten der Spitzenamplituden des Spektrums für Schmelz und Dentin. Wenn die gemessene Spitzenamplitude des Spektrums des akustischen Impulses im Frequenzband von 85 bis 95 kHz S die Bedingung: $S < (S_S + S_D)/2$ erfüllt, wobei $S_S$ und $S_D$ die Eichwerte der Spitzenamplitude des Spektrums der bei der Laserzerstörung von Schmelz bzw. Dentin auftretenden akustischen Impulse darstellen, so wird das zu bearbeitende Zahnhartgewebe als Schmelz und im gegenteiligen Fall als Dentin bestimmt.

Um den Typ des zu bearbeitenden Zahngewebes zu präzisieren, registriert man mittels der Photoregistriereinheit den Laserimpuls und fixiert den Zeitpunkt seines Beginns, mißt man die Verzögerung zwischen dem Beginn des Laserimpulses und dem Beginn des bei der Zerstörung von Gewebe entstehenden akustischen Impulses. Diese Verzögerung vergleicht man mit den Eichwerten der Verzögerungen von Schmelz und Dentin. Wenn die gemessene Verzögerung des akustischen Impulses hinsichtlich des Laserimpulses $\tau$ die Bedingung: $\tau > (\tau S + \tau D)/2$ erfüllt, wobei $\tau S$ und $\tau D$ die Eichwerte der Verzögerungen der bei der Laserzerstörung von Schmelz bzw. Dentin entstehenden akustischen Impulse darstellen, so bestimmt man das zu bearbeitende Zahnhartgewebe als Schmelz und im gegenteiligen Fall als Dentin.

Die Sicherheit der Prozedur der Bearbeitung von Zahnhartgewebe mit Laserstrahlung wird durch die Möglichkeit sichergestellt, die Energie der Lasererzeugung in Übereinstimmung mit den Informationen über den Typ des zu bearbeitenden Gewebes zu steuern. Um Informationen über den Typ des zu bearbeitenden Gewebes zu erhalten, muß man die akustischen Impulse, die bei der Zerstörung von Gewebe durch den Laserimpuls auftreten, registrieren. Bei der Bearbeitung von Gewebe treten jedoch akustische Impulse auf, die mit der Zerstörung von Gewebe nicht unmittelbar in Zusammenhang stehen. Deren Natur stellt sich wie folgt dar:

Unter der Einwirkung eines Laserimpulses wird das zu bearbeitende Zahnhartgewebe auf hohe Temperaturen erhitzt. Es erfolgt dessen teilweise Zerstörung und Verdampfung. Die erhitzten Gase bringen der Laserstrahlung unter hohem Druck Gewebeteilchen entgegen. Diese Teilchen verteilen sich mit Überschallgeschwindigkeit in der Luft, wodurch eine akustische Druckwelle entsteht. Die Amplitude der Druckwelle wird durch die Geschwindigkeit und die Größe der Teilchen bestimmt, ist unabhängig vom Typ des zu bearbeitenden Gewebes und übersteigt um ein Vielfaches (ungefähr um das 10-fache) die Amplitude des nützlichen akustischen Impulses und macht dessen Auswertung relativ problematisch. Diese Druckwelle hat jedoch, wie sich herausgestellt hat, im Vergleich zur Schallwelle, die bei der Zerstörung von Gewebe auftritt, Besonderheiten aufzuweisen. Die Druckwelle ist gerichtet und breitet sich nur in einem schmalen Raumwinkel in einer Richtung, die der Laserstrahlung entgegengesetzt ist (d.h. entlang der optischen Achse) aus. Auf diese Art und Weise ist die mit der vorliegenden Erfindung vorgeschlagene Konstruktion der Vorrichtung, die einen akustischen Empfänger enthält, der derart installiert ist, daß die Richtung seiner maximalen Empfindlichkeit mit der Richtung der optischen Achse am Austritt des Strahlungstransportmittels vom Laser zum Zahn den Winkel $\alpha$ bildet, der die Bedingung: $11° < \alpha < 86°$ erfüllt, eine sehr vorteilhafte Ausgestaltung, um die Lösung der gestellten Aufgabe zu verbessern. Das Richtungsdiagramm des akustischen Empfängers ist in den Fig. 3 - 5 als gestreckte Ellipse angedeutet.

Gemäß der Erfindung enthält die Vorrichtung (s. Fig. 3) den Impulslaser 1, dessen Austritt optisch mit dem Strahlungstransportmittel 2 vom Laser zum Zahn verknüpft ist, das hintereinander angeordnet das Stück optischer Faser 3 und das Handstück 4 enthält. Der akustische Empfänger 5 ist derart installiert, daß die Richtung seiner maximalen Empfindlichkeit mit der Richtung der optischen Achse am Austritt des Strahlungstransportmittels 2 vom Laser 1 zum Zahn einen Winkel bildet, der das genannte Verhältnis erfüllt, und daß der Austritt des Empfängers 5 durch den Spitzendetektor 6 elektrisch mit der Anzeigevorrichtung 7 verknüpft ist.

Gemäß einer anderen Ausgestaltung der Erfindung enthält die Vorrichtung zusätzlich (s. Fig. 4) eine Einheit 8 zur Spektralumwandlung und Filterung mit einem Durchlaßbereich von 85 - 95 kHz, die derart installiert ist, daß ihr Eintritt elektrisch mit dem Austritt des akustischen Empfängers 5 verknüpft ist und deren Austritt elektrisch mit dem Eintritt des Spitzendetektors verknüpft ist.

Gemäß einer weiteren Ausgestaltung der Erfindung enthält die Vorrichtung zusätzlich (s. Fig. 5) eine Einheit 9 zur Messung von Zeitintervallen und eine Photoregistriereinheit 10, wobei der Eintritt der Photoregistriereinheit 10 optisch mit dem Austritt des Lasers 1 verknüpft ist und deren Austritt elektrisch mit einem der Eintritte der Einheit 9 zur Messung von Zeitintervallen verknüpft ist, deren zweiter Eintritt elektrisch mit dem Austritt des akustischen Empfängers 5 verknüpft ist und deren Austritt elektrisch mit dem Eintritt der Anzeigevorrichtung 7 verknüpft ist.

Ein Beispiel für die konkrete Realisierung einer erfindungsgemäßen Vorrichtung stellt sich wie folgt dar:

Als Strahlungsquelle wurde der Impulslaser 1 als "ISGG: Cr, Er" ausgewählt. Das Strahlungstransportmittel 2 vom Laser zum Zahn ist in Form eines Stücks Saphirfaser 3 und eines Handstücks 4, das ein Fokussiersystem mit einer Brennweite vom 25 mm enthält, die miteinander optisch verknüpft sind, ausgeführt, wobei der optische Eintritt des Strahlungstransportmittels optisch mit dem Laseraustritt verknüpft ist. In einem Abstand von 45 mm vom Bildbrennpunkt des im Handstück befindlichen Fokussiersystems ist ein Mikrophon 5 (Marke B&K 4138) angeordnet, das in der Weise installiert ist, daß die Richtung seiner maximalen Empfindlichkeit mit der Richtung der optischen Achse am Austritt des Strahlungstransportmittels vom Laser zum Zahn einen Winkel $\alpha$ von 28° bildet. Der Austritt des Mikrophons ist elektrisch durch den Spitzenamplitudenmesser der elektrischen Impulse (Spitzendetektor 6) mit der Anzeige 7, für die ein Digitalvoltmeter verwendet wurde, verknüpft.

Die Vorrichtung enthält zusätzlich eine Einheit 8 für die Spektralumwandlung und die Filterung der elektrischen Impulse, die die rasche Fourier-Transformation ausnützt, auf Basis eines Mikroprozessors (INTEL 386) realisiert worden ist und die derart installiert ist, daß ihr Eintritt elektrisch mit dem Austritt des akustischen Empfängers 5 verknüpft ist und ihr Austritt elektrisch mit dem Eintritt des Spitzendetektors 6 verknüpft ist.

Die Vorrichtung enthält ebenfalls zusätzlich eine Photoregistrierungseinheit 10 auf Basis einer Photodiode (FD34), die optisch mit dem Austritt des Lasers 1 verknüpft ist und elektrisch mit einem der Eintritte des Zeitintervallmessers 9 auf Basis eines Digitaloszillographen (S9-16) verknüpft ist. Der zweite Eintritt des Zeitintervallmessers ist elektrisch mit dem Austritt des akustischen Empfängers 5 verknüpft. Am Austritt des Zeitintervallmessers ist die Verzögerung zwischen dem Beginn des Laserimpulses, der von der Photoregistriereinheit registriert wird, und dem Beginn des akustischen Impulses, der vom akustischen Empfänger registriert wird, in ein elektrisches Signal umgewandelt, dessen Amplitude proportional zur Größe der zeitlichen Verzögerung ist. Der Austritt des Zeitintervallmessers ist elektrisch mit der Anzeige 7 verknüpft.

Auf diese Art und Weise gestattet es die anhand von Verfahren und Vorrichtung beschriebene Erfindung auf Grundlage des Dargelegten die gestellte Aufgabe zu lösen, nämlich die Gefahr, dem Patienten bei der Laserbearbeitung von Zahnhartgewebe im Rahmen z.B. einer Kariesbehandlung oder von Zahnprothetik ein Trauma zuzufügen, herabzusetzen bzw. auszuschalten.

## Patentansprüche

1. Verfahren zur Unterscheidung der Gewebearten bei der Bearbeitung von Zahnhartgewebe mit Laserimpulsen, wobei der bei der Wechselwirkung des Laserimpulses mit dem Gewebe entstehende akustische Impuls im Bereich von 85-95 kHz registriert und hinsichtlich seiner Spitzenamplitude überprüft wird, wonach aufgrund der Höhe der Spitzenamplitude die Art des jeweils bearbeiteten Gewebes bestimmt wird.

2. Verfahren nach Anspruch 1, dadurch gekennzeichnet, daß zusätzlich zur Registrierung des akustischen Impulses die zeitliche Verzögerung zwischen dem Laserimpuls und dem zugehörigen akustischen Impuls bestimmt und zur Präzisierung der Bestimmung der bearbeiteten Gewebeart verwendet wird.

3. Verfahren zur Regelung der Strahlungsenergie bei der Bearbeitung von Zahnhartgewebe mit Laserimpulsen, wobei der bei der Wechselwirkung des Laserimpulses mit dem Gewebe entstehende akustische Impuls im Bereich von 85-95 kHz registriert und hinsichtlich seiner Spitzenamplitude überprüft wird, wonach aufgrund der Höhe der Spitzenamplitude die Art des jeweils bearbeiteten Gewebes bestimmt und die Strahlungsenergie der Gewebeart angepaßt wird.

4. Verfahren nach Anspruch 3, dadurch gekennzeichnet, daß zusätzlich zur Registrierung des akustischen Impulses die zeitliche Verzögerung zwischen dem Laserimpuls und dem zugehörigen akustischen Impuls bestimmt und zur Präzisierung der Bestimmung der bearbeiteten Gewebeart verwendet wird.

5. Verfahren nach Anspruch 3 oder 4, dadurch gekennzeichnet, daß vor dem Beginn der Bearbeitung die den unterschiedlichen Gewebearten entsprechenden Spitzenamplituden und/oder Impulsverzögerungen an Kalibriermustern bekannter Gewebearten bestimmt und als Kalibrierwerte gespeichert werden, wonach die bei der tatsächlichen Bearbeitung bestimmten Spitzenwerte und/oder Impulsverzögerungen mit den gespeicherten Kalibrierwerten verglichen und demnach die Strahlungsenergie angepaßt wird.

6. Vorrichtung zur Bearbeitung von Zahnhartgewebe mit Laserimpulsen, mit einem Impulslaser (1) und einer eingangsseitig mit dem Laseraustritt in Verbindung stehenden Strahlungstransporteinrichtung (2), wobei ein akustischer Empfänger (5) in der Nähe des zu bearbeitenden Bereiches so angeordnet ist, die Richtung seiner maximalen Empfindlichkeit mit der Richtung der optischen Achse am Austritt der Strahlungstransporteinrichtung (2) einen Winkel $\alpha > 11°$ und $< 86°$ bildet, der elektrische Signalausgang des Empfängers (5) mit dem Eingang eines Spitzenamplitudenmessers (6) verbunden ist, dessen Ausgang mit einer Anzeigevorrichtung (7) in Verbindung steht, und wobei dem Spitzenamplitudenmesser (6) eine Einheit (8) zur Spektralumwandlung und Filterung der vom akustischen Empfänger (5) gelieferten elektrischen Signale vorgeschaltet ist, welche einen Durchlaßbereich von 85 - 95 kHz aufweist.

7. Vorrichtung nach Anspruch 6, wobei am Laseraustritt eine Photoregistriereinheit (10) optisch eingekoppelt ist, deren elektrischer Signalausgang mit einem Eingang einer Intervallmeßeinheit (9) verbunden ist, deren anderer Eingang mit dem Signalausgang des akustischen Empfängers (5) verbunden ist und deren Ausgang mit der Anzeigevorrichtung (7) in Verbindung steht.

8. Vorrichtung nach Anspruch 6 oder 7, wobei der akustische Empfänger (5) in einem am Ausgang der Strahlungstransporteinrichtung (2) angeordneten Handstück (4) integriert ist.

## Claims

1. Process for distinguishing kinds of tissues in the treatment of hard tooth tissue with laser pulses, the acoustic pulse in the range from 85-95 kHz arising on the interaction of the laser pulse with the tissue being registered and being tested in respect of its peak amplitude, whereby the nature of the tissue undergoing treatment is identified on the basis of the height of the peak amplitude.

2. Process according to claim 1, characterised in that in addition to the registration of the acoustic pulse the time delay between the laser pulse and the associated acoustic pulse is determined and is used to make more precise the determination of the kind of tissue being treated.

3. Process for regulating the radiation energy in the treatment of hard tooth tissue with laser pulses, the acoustic pulse in the range from 85-95 kHz arising on the interaction of the laser pulse with the tissue being registered and being checked with regard to its peak amplitude, whereby on the basis of the height of the peak amplitude the nature of the tissue currently undergoing treatment is determined and the radiation energy is matched to the kind of tissue.

4. Process according to claim 3, characterised in that in addition to the registration of the acoustic pulse the time delay between the laser pulse and the associated acoustic pulse is determined and is used for making more precise the determination of the kind of tissue being treated.

5. Process according to claim 3 or 4, characterised in that before the start of the treatment the peak amplitudes and/or pulse delays corresponding to the different kinds of tissue are determined on calibrating samples of known kinds of tissue and are stored as calibrating values after which the peak values and/or pulse delays determined in the actual treatment are compared with the stored calibrating values and the radiation energy is matched accordingly.

6. Apparatus for treatment hard tooth tissue with laser pulses, with a pulse laser (1) and a radiation transporting device (2) connected at its input to the output of the laser, in which an acoustic pick-up (5) is arranged in the neighbourhood of the region to be treated in such a way that the direction of its maximum sensitivity makes an angle $\alpha > 11°$ and $< 86°$ with the direction of the optical axis at the output of the radiation transporting device (2), the electrical signal output of the pick-up (5) is connected to the input of a peak amplitude measuring device (6) of which the output is connected to a display device (7), and in which the peak amplitude measuring device (6) has connected ahead of it a unit (8) for spectral conversion and filtering of the electric signals delivered by the acoustic receiver (5), having a pass band of 85-95 kHz.

7. Apparatus according to claim 6, in which a photoregistering unit (10) is optically coupled into the output of the laser and has its electric signal output connected to one input of an interval measuring unit (9), of which the other input is connected to the signal output of the acoustic pick-up (5), and of which the output is connected to the display device (7).

8. Apparatus according to claim 6 or 7, in which the acoustic pick-up (5) is integrated into a handpiece (4) arranged at the output of the radiation transporting device (2).

## Revendications

1. Procédé pour différencier des types de tissus au cours du traitement de tissus dentaires durs par des impulsions laser, dans lequel on enregistre l'impulsion acoustique produite durant l'interaction de l'impulsion laser avec les tissus dans la zone 85-95 kHz, et on l'examine quant à son amplitude de pointe, et ensuite on détermine le type de tissu respectif traité à partir du niveau de l'amplitude de pointe.

2. Procédé selon la revendication 1, caractérisé en ce que, en plus de l'enregistrement de l'impulsion acoustique, on détermine le retard temporel entre l'impulsion laser et l'impulsion acoustique associée, et que l'on utilise pour déterminer avec plus de précision le type de tissu traité.

3. Procédé pour régler l'énergie de radiation au cours du traitement de tissus dentaires durs par des impulsions laser, dans lequel on enregistre l'impulsion acoustique produite pendant l'interaction de l'impulsion laser avec le tissu dans la zone 85-95kHz, et on l'examine quant à son amplitude de pointe, et ensuite on détermine dans chaque cas le type de tissu respectif traité, à partir du niveau de l'amplitude de pointe, et on adapte l'énergie de radiation au type de tissu.

4. Procédé selon la revendication 3, caractérisé en ce que, en plus de l'enregistrement de l'impulsion acoustique, on détermine le retard temporel entre l'impulsion laser et l'impulsion acoustique associée et on l'utilise pour déterminer avec plus de précision le type de tissu traité.

5. Procédé selon la revendication 3 ou 4, caractérisé en ce qu'on détermine, avant le début du traitement, les amplitudes de pointe et/ou les retards d'impulsion correspondant aux divers types de tissu à partir de modèles de calibrage, et on met en mémoire ces amplitudes et retards comme valeurs de calibrage, on compare ensuite les valeurs de pointe et/ou les retards d'impulsion déterminés au cours du traitement réel, avec les valeurs calibrées mises en mémoire, et on adapte ensuite l'énergie de radiation.

6. Dispositif pour traiter des tissus dentaires durs par des impulsions laser, comprenant un laser à impulsion (1) et un appareil de transport de radiation (2) se trouvant en liaison avec la sortie laser du côté entrée, dans lequel un récepteur ou capteur acoustique (5) est disposé à proximité de la zone à traiter de telle façon que la direction de sa sensibilité maximale fasse avec la direction de l'axe optique à la sortie de l'organe (2) de transport de radiation, un angle $\alpha > 11°$ et $< 86°$, dans lequel la sortie du signal électrique du récepteur (5) est reliée à l'entrée d'un appareil de mesure (6) de l'amplitude de pointe dont la sortie est reliée à un dispositif d'affichage (7), et dans lequel, en amont de l'appareil de mesure de l'amplitude de pointe, est branchée une unité (8) pour la conversion spectrale et le filtrage des signaux électriques émis par le récepteur acoustique (5) présentant un domaine de transmission de 85 à 95kHz.

7. Dispositif selon la revendication 6, dans lequel, à la sortie laser, est couplée optiquement une unité d'enregistrement photographique (10) dont la sortie des signaux électriques est reliée à l'entrée d'une unité de mesure d'intervalles (9), dont l'autre entrée est reliée à la sortie (10) de signaux du récepteur acoustique (5) et dont la sortie est reliée au dispositif d'affichage (7).

8. Dispositif selon la revendication 6 ou 7, dans lequel le récepteur acoustique (5) est intégré dans un outil à main (4) placé à la sortie de l'appareil de transport de radiation (2).

Fig. 1a

Fig. 1b

Fig. 1c

Fig.2a

Fig.2b

Fig.2c

Fig.2d

Fig. 3

Fig. 4

Fig. 5